# EUROPEAN PATENT APPLICATION

(11) **EP 1 057 490 A2**
(43) Date of publication of application: **06.12.2000**
(21) Application number: 00500101.1
(22) Date of filing: 25.05.2000
(51) Int. Cl.: A61K 38/38, A61K 38/36, A61K 31/19

(54) **Use of tranexamic acid for the preparation of a human fibrinogen composition**

(30) Priority: 31.05.1999 ES 9901175
(71) Applicant: Grupo Grifols, S.A., 08150 Parets Del Valles, Barcelona (ES)
(72) Inventor: Debart, Pere Ristol, Sabadell (Barcelona) (ES); Camison, Isabel Bravo, 08240 Manresa (Barcelona) (ES); Rodriguez, Jesus Fernandez, 08850 Gavá (Barcelona) (ES)
(74) Representative: Duran Moya, Luis-Alfonso

(57) **Abstract**

Use of tranexamic acid for the preparation of a human fibrinogen composition.

The invention relates to the use of tranexamic acid for the preparation of a composition of human fibrinogen obtained from human plasma or by methods of recombinant or transgenic technology, with the simultaneous addition of human albumin, in order to reduce the solubilisation time when a physiologically compatible solvent is added, and relates also to the fibrinogen composition.

## Description

The present invention relates to the use of tranexamic acid for the preparation of a composition of human fibrinogen obtained from human plasma or by methods of recombinant or transgenic technology, with the simultaneous addition of human albumin, in order to reduce the solubilisation time when a physiologically compatible solvent is added. The composition is dried and virus-inactivated by heat. A solution of this preparation may be applied topically as an adhesive or a biological glue in order to join human organs or tissues.

Protein preparations of this type which are found on the market comprise antifibrinolytic agents as auxiliary protectors of the fibrin clot, such as aprotinin (of animal origin) or, for example, e-aminocaproic acid (of synthetic origin) or the like. The need to add those exogenous compounds to the basic coagulable fibrinogen component has been sufficiently discussed and disputed, owing to the lack of clinical results supporting this need when the fibrinogen is used as a biological adhesive.

Furthermore, the current state of the art includes, as the most relevant items of prior art connected with the process of the invention, those which are discussed below:

The use of antifibrinolytic agents, such as aprotinin, goes back to the 1970s (aprotinin is currently marketed under the registered names of: Antagosan, Fase, Iniprol, and others). Administration together with fibrinogen, for its application as a biological glue, has already been disclosed in patent DE 3002933 (priority 15/02/79) in which specific mention is made of the properties of the fibrin adhesive formed and of the stability conferred by the antifibrinolytic agent.

A readily solubilisable lyophilised composition formulated with amino acids containing urea or guanidine groups in their molecule is described in patent ES 519469 (priority 04/02/82).

Much later, compositions were described which improve the solubility of the dehydrated fibrinogen preparation and/or its stability in solution by the previous addition of urea (patent WO 91/19519 with priority 15/06/90) and by the presence of nonionic detergents (patent ES 2080155 with priority 01/08/89, and patent WO 95/25748 with priority 18/03/94).

Various patents have been applied for which relate to specific or nonspecific methods of virus inactivation for fibrinogen. Patent ES 536850 (priority 19/10/83) claims virus inactivation by the thermal treatment of proteins, including fibrinogen, in the dried state and in the presence of acidic and basic amino acids. Patent ES 524992 (priority 19/08/82) also relates to the process of virus inactivation by pasteurisation in liquid at 60°C for 10 hours and in the presence of stabilisers.

Patent ES 2051914 (priority 29/04/88) describes the composition and preparation of a compound which can be used for repairing cartilage and bone and which is constituted by a biodegradable biological glue, the form of preparation of which was described at an earlier date in patent US 4642120 (priority 28/03/83). The gel claimed in the patent in question, ES 2051914, or in previous, US 4642120, is constituted, inter alia, by thrombin and calcium chloride, antiprotease (aprotinin, epsilon aminocaproic acid, tranexamic acid, or other protein inhibitors, such as antiplasmin, etc.) and fibrinogen. According to the mentioned patent, the compound can be stored for 2-3 days at incubation temperatures, or for 2-3 years at cryogenic temperatures. The application claimed in that patent relates solely to the use of bone marrow cells together with the immobilisation mediator (adhesive glue), which contains not less than 10% of serum.

Patent WO 92/22312 (priority 17/06/91) specifically claims a composition having adhesive properties which comprises biodegradable fibrinogen or fibrin and biocompatible polymer, so that it is basically an adhesive glue which maintains, at the very least, the known clinical applications of the standard preparations of these types of compound. The use of some of the already known antifibrinolytic agents of human origin (antiplasmin), animal origin (aprotinin) and synthetic origin (tranexamic acid) is mentioned for the preparation thereof.

Another of the patents relating to biological glues is EP 0534178 A2 (priority 27/09/91). This patent describes the preparation of an adhesive based on a compound A of crude fibrinogen and another compound B preferably constituted by a proteolytic enzyme obtained from snake venom (batroxobin) or by thrombin. This patent considers the possibility of adding a protease inhibitor, such as aprotinin. For the preparation of compound A, it describes exclusively the use of cryoprecipitate as starting material, stating that the composition of that material (pool of 5 plasma donations) is: total protein 75 mg/ml (100%), fibrinogen 36 mg/ml (48% relative to total protein) and factor XIII 4.10 IU/ml. According to the description of the patent, the processed product, that is to say, after extraction with citric saline solution and filtration in the presence of aluminium gel, has a protein concentration equivalent to the initial concentration (70-80 mg/ml of total protein), being kept frozen in a plastics bag (lyophilisation is regarded solely as optional).

Patent WO 94/22503 (priority 30/03/93) describes the production and application of an adhesive fibrin glue, the advantages of which indicated in the invention reside in the greater mechanical strength of the adhesive glue of the preparation. Explicit mention is made of tranexamic acid as a substitute for aprotinin, having antifibrinolytic activity of low immune response, capable of conferring sufficient tensile strength when compared with other preparations containing aprotinin. In no case is there any mention of the use of tranexamic acid or its derivatives as excipients of fibrinogen during lyophilisation, alone or in combination with other components, or of its marked solubilising effect, discovered by the inventors, which is presented in this specification. Some of the characteristics of the preparations of the type claimed in the patent in question have been published (Jackson M.R., MacPhee M.J., et al. Fibrin Sealant: Current and Potential Clinical Applications. Blood Coagulation and Fibrinolysis; 7, 737-746; 1996).

On the other hand, both the chemical synthesis and the therapeutic indications of tranexamic acid (antifibrinolytic activity) by the intravenous, subcutaneous, oral or topical route (ointments and solutions) are reported in Belgian patent No. 617216 (priority 1961), which emphasises the specific activity of the preparation with regard to its inhibiting effect on the presence of plasmin, with respect to the lysis of the fibrin clot formed by fibrinogen and thrombin, in the presence of Ca(2+) ions. That patent also claims the possible use of the product combined with other active principles or ingredients, the nature of which promotes fibrinolytic activity.

This novel composition of the preparation disclosed in the invention solves several of the problems which are currently exhibited by coagulable fibrinogen compounds for topical use, and which consist in:
1.- The use of compounds having a low concentration of coagulable proteins which have an effect on the activity of the preparation, compromising its therapeutic usefulness.
2.- Nowadays, physical and/or chemical methods of inactivation-sterilisation confer on products derived from human plasma a practically zero risk of infection by the majority of the most dangerous pathogenic viruses transmittable by plasma, such as those which cause human immunodeficiency and hepatitis B or C (and very possibly G) or any of the known viruses having a lipid envelope. However, there are no absolutely sure methods for the complete sterilisation of viruses without a lipid envelope transmittable by plasma.
3.- The production of preparations of coagulable fibrinogen which are preserved in the dry (lyophilised) state and are inactivated by heat and which are readily solubilised in an aqueous carrier and at high concentration, continues to be a factor limiting the topical application of the preparation with maximum therapeutic efficiency.

The points indicated above have been dealt with by the authors of the present invention who have provided a composition of an adhesive protein complex which is superior to current equivalent products of the prior art.

For the topical use of the protein complex to which the invention relates as a biological adhesive, an auxiliary compound is required to form a clot of adhesive fibrin. The auxiliary compound, constituted by an activated enzyme, such as thrombin, gives rise to a strong fibrin lattice when brought into contact with the protein complex of the invention, in the presence of calcium ions.

One of the principal proteins forming the preparation of the invention is coagulable fibrinogen. That majority protein is accompanied by other adhesive proteins which, in the case of topical application as a glue, are especially valuable since they participate in the mechanisms of joining tissue by conferring improved adhesion. Thus, this protein complex is characterised in that, in addition to containing predominantly fibrinogen, it is accompanied by fibronectin and also by factor XIII and other minority proteins.

The concentration of coagulable fibrinogen is one of the subjects of interest in the present invention. It is known that both the speed of formation of the fibrin lattice and its strength are a function of the concentration of the coagulable protein in the protein complex applied. It is therefore a vital requirement to administer topically a high concentration of fibrinogen so that, when brought into contact with its activator, it gives rise to a strong lattice of cross-linked fibrin.

The present invention deals with the problems of preserving the preparation under conditions which do not require freezing temperatures, so that it can be stored at ambient temperature or in a refrigerator, for which purpose a formula has been specially developed which enables a lyophilised preparation to be obtained which fulfils pharmacopoeia requirements and which is superior from that point of view to several of the preparations that have been registered and marketed or that are still in the development stage.

Another of the especially relevant points of the present invention is the incorporation of at least one specific viral inactivation step of recognised effectiveness. This inactivation is effected by high-temperature terminal thermal treatment in the dried state (brought about by lyophilisation, for example) and in the finished product in its final packaging. The fibrinogen compound so treated maintains its biological and physical properties unaltered.

In order to obtain a preparation of adhesive proteins having the characteristics indicated in the above points, the inventors have developed an optimum composition for its topical use as a biological glue.

The monograph of the European Pharmacopoeia for the Fibrin Adhesive indicates that the coagulable fibrinogen component must contain not less than 60 g/litre, and also not less than 10 IU of solution factor XIII/ml, and the resolubilisation time of the lyophilised product must not be more than 20 minutes at the preferred temperature indicated.

The above-mentioned fibrinogen concentration is not readily achievable in compositions of preparations obtained by simple processes for the production of adhesive protein complexes, for example starting from a cryoprecipitate, owing to the large amount of accompanying proteins which contaminate the basic fibrinogen preparation. However, and even if it were possible to achieve the necessary concentration of fibrinogen by those methods, the material obtained is preferably kept in the frozen state until it is used. The dried (lyophilised) preparations are poorly solubilisable, within a reasonable period of time, at the concentrations required for the use thereof as a biological tissue adhesive, so that the solution is preserved in the frozen state (applied to "home-made" preparations).

At present, it continues to be common practice to prepare fibrinogen compounds starting from a cryoprecipitate of autologous plasma, from a single donor or from a mixture of very few donors (for example, 5). These types of preparation ("home-made" or in-house preparations) are produced by hospital centres for their own use (internal use), but they exhibit a clear lack of standardisation and reproducibility with regard to the quality of the preparation and therefore inaccurate results and low therapeutic efficiency are obtained. Nor does this practice completely exclude the risk of viral transmission (except in the case of autologous plasma) and very low final concentrations of fibrinogen are obtained, ranging approximately from 10 to 29 mg/ml (1% - 2.9%) of coagulable fibrinogen (Jackson M.R., MacPhee M.J., et al. Fibrin Sealant: Current and Potential Clinical Applications. Blood Coagulation and Fibrinolysis; 7; 737-746; 1996).

Likewise, the use of the above-mentioned fibrinogen preparation could have a pronounced negative effect owing to one of the remaining accompanying proteins (it presumably contains only approximately 50%, and in any case less than 70%, of coagulable fibrinogen) and it may therefore be practically indispensable to add a protease inhibitor providing stability and protection first to the fibrinogen and then to the fibrin lattice formed when it is applied topically.

The solubility of the lyophilised preparation has been improved by the technique of incorporating amino acids containing a urea or guanidine group, such as arginine, isoleucine, etc., or by chaotropic agents, such as urea itself. A description has also been given of the use of soluble fatty acids and detergents which, according to what has been published, may promote the solubilisation of the lyophilisate.

In any case, it is necessary to maintain conditions of approximately physiological ionic strength at the time of forming the fibrin lattice. It is regarded as unacceptable to prepare solutions of which the final ion concentration clearly exceeds the physiological value (for example, more than double that value).

The inventors have solved the problem of maintaining the stability of a fibrinogen preparation containing the majority of the adhesive proteins of interest and factor XIII, preserved in a lyophilised state, creating the possibility of virus inactivation by heat in the dry state, with the purpose of providing the coagulable proteins in high concentration.

Surprisingly, the inventors have discovered a final composition or formula which, in optimum quantities of the excipients, permits the solubilisation of the lyophilised fibrinogen compound at concentrations higher than 60 g/litre of coagulable protein, the time taken for its reconstitution being clearly lower than the mandatory 20 minutes for the solubilisation of the lyophilised product, and being generally from 5 to 10 minutes. The formula of the invention permits efficient virus inactivation by heat at high temperature, without damaging the functional character of the preparation, and, in the same way, it maintains its physical properties without significant change.

With regard to the generation of an immune response by the preparation, this may be practically zero in the infusion of compounds having a high level of purification, in which the presence of immunoglobulins (isoaglutinins) has also been sufficiently reduced. The possible formation of neoantigens, which is due principally to the action of heat during the final stage of thermal inactivation of the lyophilised product, could be practically excluded if the degree of drying of the lyophilised product and the favourable atmosphere (absence of oxygen) for heating the solid were controlled efficiently.

With respect to the already indicated viral safety of the fibrinogen compounds, there is currently a practically zero risk for viruses having a lipid envelope if the preparations have been subjected to one of the recognised methods of inactivation (solvent-detergent). Historically, the fibrinogen used in the 1970s or before exhibited a very high risk of transmitting viral diseases, caused principally by hepatitis (B, and/or non-A, non-B), if no specific virus inactivation step was included in its production, when a pool of plasma from hundreds or thousands of donors was used as the starting material. There was therefore a need to prepare fibrinogen from autologous plasma, or from a very small and controlled number of donors.

At present, plasma fractionation companies market compounds of virus-inactivated coagulable fibrinogen, with at least one highly efficient and specific step of virus elimination (it is estimated that it is necessary to have at least an approximately 8-10 Log(10) reduction in the initial viral charge in the total of the production process, for viruses having a lipid envelope, and at least one step in which the residual infectiousness is zero). Thus, the fibrinogen products which are currently being developed should include a double inactivation step in which at least one of those steps is also effective towards viruses without a lipid envelope, in order to confer a sufficient level of safety on the product.

The process of the invention meets the challenge of dual inactivation with the possibility of combining the chemical inactivation (solvent-detergent) introduced in the process for purifying the product, with high-efficiency thermal inactivation in the final packaging, these being complementary and selective.

It should be pointed out that viral inactivation by means of heat in the dry state may easily act against the speed of solubilisation of the lyophilised product so treated and against the quality of the solution obtained. By means of a novel lyophilised formulation, the process of the invention overcomes that key difficulty to obtain a suitable fibrinogen preparation which takes into account inactivation by heat.

The inventors discovered that a suitable combination of proteins, amino acids and chemical reagents was able to promote the solubilisation of dehydrated material. Surprisingly, they found that the adhesive complex of fibrinogen suitably mixed with albumin, glycine and sodium citrate salts, to which was added at least one solubiliser from the group formed by an amino-substituted carboxylic acid having a non-linear hydrocarbon chain, at a precise concentration, led to a preparation which amply meets the specifications of the monograph of the product for its preservation in the dried state.

The solubiliser in question corresponds more specifically to the formula of a chemically synthesised compound which is formed by a carboxylic acid group linked to an aliphatic cyclic ring having 6 carbon units and likewise substituted by a terminal aminomethyl group in the 4 or "para" position. The semi-developed formula corresponds to the expression: NH₃CH₂ - A - CO.OH; where A is a cyclohexane ring. The compound referred to is 4-aminomethylcyclohexanecarboxylic acid which is known by the abbreviation AMCHA (or also as AMCA) of which there are two position isomers, cis-AMCHA and trans-AMCHA, the latter having antifibrinolytic activity, and which is also known as tranexamic acid, Cyklokapron or by other names (with patent application US 3499925, priority 1965).

Surprisingly, the inventors have now discovered, and describe in this specification, that this compound acts as a solubiliser of fibrinogen (and accompanying adhesive proteins) in the dried (lyophilised) state, when it forms part of a suitable combination of other components (albumin, glycine, salts). If one of the above-mentioned components is absent, whether albumin or glycine or salts, the solubilising effect of the AMCHA added is markedly reduced.

In order to achieve the desired aim with respect to the solubilisation of the product, the inventors carried out a thorough experimental investigation to find out what compositions might be acceptable, both qualitatively and quantitatively, so that it was possible to achieve optimisation thereof.

The composition of the product of the invention has the enormous added advantage that it permits the application of a virus inactivation step by heat at elevated temperature, which confers a maximum level of viral safety when it is combined with chemical inactivation by solvent/detergent. The excipients or stabilisers of the composition are heat-resistant under the heat-treatment conditions. The albumin used in the formulation participates by stabilising the product during the heating operation, and the substituted carboxylic acid continues by facilitating the solubilisation of the lyophilised product after that treatment.

A detailed description of the composition and the preparation process of the invention is given below:

The protein solution of the starting material, which solution is purified and concentrated to approximately 4% of total protein, of which at least 90% is coagulable fibrinogen, is in an aqueous medium which contains a glycine concentration of from 0.05M to 0.2M, a sodium chloride concentration of from 0.05M to 0.2M and a sodium citrate concentration of from 0.01M to 0.05M, at a pH of preferably from 6.0 to 8.0.

The starting product is practically free from high-molecular-weight fibrinogen aggregates and also from unstable lipoprotein compounds or other compounds that may be altered by lyophilisation, or by some other type of drying operation.

From 0.25% to 0.75% of human serum albumin is added to the solution of the product, followed by a concentrated solution of a compatible solubiliser, constituted by a substituted cyclic carboxylic acid and preferably by 4-aminomethylcyclohexanoic acid (AMCHA), obtaining a concentration of preferably from 1% to 4%, and more preferably from 2% to 2.5%, once the solution has been adjusted to a concentration of 3%-4% of fibrinogen.

Before being dried (lyophilised, atomised), the adjusted solution is purified and sterilised by filtration with up to 0.2-micron pores, measuring out the appropriate volume required for the desired presentation, and preferably measuring out a volume equivalent to double that of the intended reconstitution, for example 10 ml of a 3-4% fibrinogen solution which is reconstituted with only 5 ml of solvent (water for injection).

The product is dried preferably by a suitable lyophilisation cycle (preferably by preliminary freezing of the product at temperatures of some -60°C or lower, and final drying at some +30-40°C under high vacuum), or by atomisation, the product then being subjected to a thermal heating step effective in inactivating heat-sensitive viruses, at a high temperature of ≥ 80°C-115°C and with a period of exposure of preferably from 5 minutes to 100 hours, in its sealed final packaging and under vacuum.

The lyophilised fibrinogen compound intended for use as an adhesive glue, once virus-inactivated by heat, is dissolved preferably in water for injection, using a volume of solvent such that the final fibrinogen concentration is not less than 60 mg/ml, and is preferably from 60 to 80 mg/ml. The content of coagulable fibrinogen in relation to the total protein is greater than 70%. Likewise, the concentration of the excipients once the dehydrated product has been solubilised is: sodium chloride preferably from 0.1 M to 0.4M, sodium citrate preferably from 0.02M to 0.1 M, glycine preferably from 0.1M to 0.4M, human albumin preferably from 0.5% to 1.5%, and the concentration of the substituted cyclic carboxylic acid (AMCHA) is preferably from 2% to 8% and more preferably from 4% to 5%. The solubilisation time of the product under rotational agitation is less than 20 minutes, and is generally from 2 to 10 minutes if the product is not subjected to thermal treatment and preferably from 2 to 15 minutes once it is heated under the preferred conditions described above. The temperature at which the lyophilisate is dissolved may be ambient temperature (approximately 22°C) or higher, but must not exceed 40°C. Preferably, the dry solid is dissolved with water at a temperature of from 35 to 37°C, the container (vial) which contains the suspension of the product being subjected to moderate and constant agitation which may be provided by any means, whether manual or mechanical (by rotation), until dissolution is complete. The solution obtained is transparent and colourless or has a slightly yellowish shade.

The addition of an activator (thrombin) to the adhesive fibrinogen compound of the invention promotes rapid activation with the formation of a resistant and stable biologically compatible and adsorbable fibrin clot which may be used as a tissue adhesive with the clinical indications characteristic of this type of preparation.

The process to which the present invention relates will be indicated below by means of a schematic diagram.

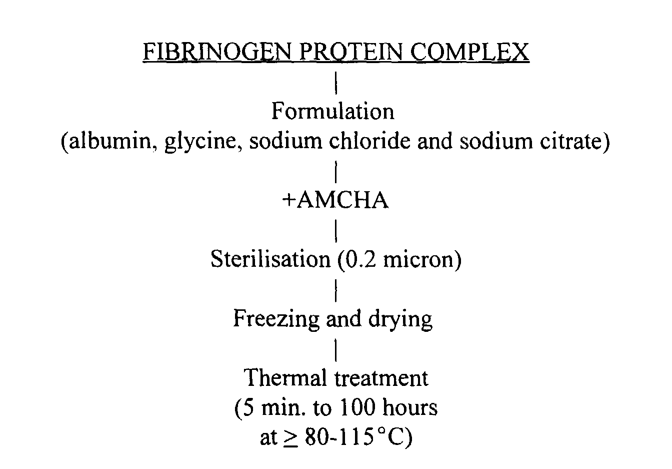

### EXAMPLES

### Example 1

A processing batch (no. 7006) for obtaining fibrinogen (protein complex), purified by precipitation with glycine, was processed to give a lyophilised end product for possible topical application as a biological glue.

The starting material was 474 g of a precipitate of fibrinogen with glycine, suspended in 1422 g of the solution for solubilisation. The 1422 g of solution for solubilising the precipitate contained 10.69 g of sodium chloride, 8.36 g of trisodium citrate dihydrate and 45.504 g of sucrose. The precipitate was maintained under agitation for not less than 45 minutes and at a temperature of some 30°C. After readjusting the pH of the suspension to 6.6 with 1 molar acetic acid, the suspension was centrifuged in a Sorvall-brand vessel centrifuge, model RC-3, at a rate of 4000 rpm and at a temperature of some 23°C. The weight of precipitate obtained was 11.6 g. The centrifugation supernatant exhibited a turbidity of 106 NTU and an optical density at 280 nm of 49.9 UA. The above supernatant was then purified by filtration using CP-20 (Millipore brand) to give 1730 g of filtrate, the optical density of which at 280 nm was 49.6 UA and the turbidity of which was 103 NTU.

Diafiltration was then carried out using ultrafiltration equipment with 3 100-kDa cassettes of nominal molecular section and 15 ft² total surface area, constructed with polyether sulphone having a low capacity for protein adsorption (Omega series of the Pall-Filtron brand). The ultrafiltration conditions were the usual ones for this type of operation, the supply pressure being from 1.0 to 1.4 bar and the retained portion being lower than 0.5 bar. The rate of flow of filtrate at some 23 °C was approximately 150 ml/minute at the start of the operation. The solution was subjected to diafiltration by adding fractions, up to 6 volumes of dialysis solution being used, by the technique of addition and reduction of volume. The dialysis solution was composed of 0.124 molar sodium chloride and 0.02 molar sodium citrate, at a pH of 6.5-6.7.

Finally, the solution of the product had a concentration such that its optical density at 280 nm was 62.6 UA.

Different concentrations of argenine of up to 1%, 2% and 3% were added to a portion of the concentrated final solution. 3.0 ml of therapeutic albumin concentrated to 20% were added to some 240 ml of the solution. This last-mentioned solution, stabilised with albumin, was divided into 2 portions, and glycine was added to one of them up to a concentration of 0.1 M. Different concentrations of AMCHA were added to the previous 2 solutions to give a final concentration of 0%, 1% and 2%. Each of the solutions having a different composition was sterilised by filtration using a PVDF membrane (Durapore brand) having 0.22-micron pores in the form of discs 47 mm in diameter.

They were metered into vials having a nominal volume of 10 ml. The vials were frozen in a compartment at approximately -70°C and then lyophilised under high vacuum, the final drying operation being carried out at approximately 37°C for not less than 24 hours.

The lyophilised vials were resolubilised with 5 ml of water for injection at a temperature of 35-37°C, in each case obtaining a fibrinogen concentration higher than 60 mg/ml, which is reflected in the lyophilisate dissolution times in Table 1.

**Table 1**

| Composition of the preparations | | | |
|---|---|---|---|
| Arginine (%) | AMCHA (%) | Glycine (molar) | Solubilisation time (minutes) |
| 1 | 0 | 0 | 28 |
| 2 | 0 | 0 | 18;20 (n=2) |
| 3 | 0 | 0 | 20 |
| 0 | 0 | 0.1 | >20;>20;20 (n=3) |
| 0 | 1 | 0.1 | 15 |
| 0 | 2 | 0.1 | 4;4 (n=2) |
| 0 | 1 | 0 | >25;25 (n=2) |
| 0 | 2 | 0 | 12 |
| Note: All of the compositions also contained 0.124 molar sodium chloride and 0.02 molar sodium citrate, at pH 6.5-6.7. Those formulated with AMCHA contained up to 0.5% of albumin. | | | |

According to the solubilisation times of the lyophilised products with water (5 ml) at 35-37°C, the group that includes AMCHA in its composition stands out. Concentrations of AMCHA of approximately 2% give rise to solubilisation times of 4 minutes, if the formula also includes 0.1 molar glycine, in addition to sodium chloride-sodium citrate-albumin.

The results indicate that it is possible to obtain solubilisation times substantially lower than 20 minutes when AMCHA and glycine are included in the basic composition of sodium chloride, sodium citrate and 0.5% albumin. In the absence of AMCHA and/or glycine, the values of those times clearly increase.

### Example 2

With the aim of optimising the conditions of the composition of the product for the effective inactivation by heat of any viruses present in the final fibrinogen compound, various concentrations of albumin were tested in the final composition of the product, determining the recovery of fibrinogen and accompanying factor XIII.

The purified product constituting the fibrinogen complex was processed as in Example 1. Its composition was adjusted to a final concentration of 0.124 molar sodium chloride, 0.02 molar sodium citrate, 0.1 molar glycine and different amounts of albumin which led to concentrations of 0.4%, 0.6% and 0.8%. When the product had been lyophilised, it was heated at 100°C for 1 hour.

The product before and after thermal treatment was solubilised with water and characterised by quantifying the protein by Bio-Rad, and the coagulable activity of fibrinogen and factor XIII, the solubilisation time of the materials also being determined.

The results obtained are shown in Table 2.

**Table 2**

| Composition in % of albumin | Non-heated product | | | Heated Product | | |
|---|---|---|---|---|---|---|
| | Protein conc. (%) | Fibrinogen (%) | Factor XIII (IU/ml) | Protein conc. (%) | Fibrinogen (%) | Factor XIII (IU/ml) |
| 0.4 | 4.44 | 4.7 | 55.52 | 4.48 | 5.0 | 48.25 |
| 0.6 | 5.03 | 5.06 | 61.13 | 4.67 | 5.48 | 51.6 |
| 0.8 | N.D. | 5.22 | 44.18 | N.D. | 5.64 | 43.21 |
| N.D.: Not determined | | | | | | |

The solubilisation time was from 14 to 20 minutes for the non-heated product, and was in all cases more than 20 minutes after heating the product.

The recovery of fibrinogen and factor XIII in the heated product relative to its content in the previous step, corrected by its total protein content, is shown in Table 3.

**Table 3**

| Composition in % of albumin | Fibrinogen recovery (%) | Factor XIII recover (%) |
|---|---|---|
| 0.4 | 105 | 86 |
| 0.6 | 117 | 91 |
| 0.8 | 108 | 98 |

The results of the above Tables 2 and 3 on the one hand reflect a high concentration of factor XIII in the lyophilised end material, once regenerated with water to concentrations of approximately 5% of fibrinogen. In all cases, the activity of factor XIII is greater than 40 IU/ml.

On the other hand, the activity recovery values, both of fibrinogen and of factor XIII, which are produced by the thermal treatment applied, are compiled in Table 3. It may be deduced from the results that fibrinogen is sufficiently stable at any of the albumin concentrations of the composition prepared. The activity of factor XIII is largely preserved during heating, in the compositions having the albumin concentrations studied, i.e. from 0.4% to 0.8%. However, there is a fair reduction in the activity of factor XIII by heating, which correlates with the reduction in the albumin concentration in the composition.

### Example 3

Various batches of purified fibrinogen complex were processed to give the desired final formulation, and then the end product was lyophilised and solubilised.

The weight of the starting material taken from each of the three batches of purified fibrinogen complex (nos. 7006, 7007 and 7008) was approximately equal, being some 500 g in each case, and the starting materials were processed independently as described in Example 1 to obtain the lyophilised end product. The various compositions of the end product, which are indicated in Table 6, were prepared. The results obtained with regard to the dissolution time of the lyophilisate, the time taken to form the fibrin clot in the presence of thrombin (solution of some 100 IU/ml), the turbidity of the reconstituted lyophilisate and the gelling time, are shown in Table 4.

**Table 4**

| Processing batch no. | Composition (% AMCHA) | Solubilisation time (min.) (n=3) | Coagulation time (sec.) | Turbidity (NTU) (n=2) | Gelling (at 24 hours) |
|---|---|---|---|---|---|
| 7006 | 0 | >20 | 11 | 56.5±0.6 | NO |
| | 1 | 13+2 | 16 | 48.2±0.7 | NO |
| | 2 | 4+1 | 16 | 38.7±0.1 1 | NO |
| | 2.5 | 9+1 | 16 | 34.7±3.9 | NO |
| | 3 | 7+4 | 21 | 34.7±1.7 | NO |
| 7007 | 0 | >20 (*) | N.D. | 63.1(*) | NO |
| | 1 | >20 (*) | N.D. | 53.9 (*) | NO |
| | 2 | 8 (*) | N.D. | 47.9 (*) | NO |
| | 3 | 8 (*) | N.D. | 43.8 (*) | NO |
| 7008 | 0 | >20 | 9 | 79.3±0.7 | NO |
| | 1 | >20 | 12 | 59.2±0.6 | NO |
| | 2 | 13+10 | 14 | 46.4±2.9 | NO |
| | 2.5 | 9+4 | 12 | 44.6±0.9 | NO |
| | 3 | 8+3 | 13 | 42.5±2.5 | NO |

| | | | | | |
|---|---|---|---|---|---|
| (*) Single determination of 1 vial. N.D. = Not determined Note: (1) The percentage concentration of AMCHA indicated corresponds to the solution before lyophilisation, the metering and reconstitution volume being 10 and 5 ml in each case. (2) In order to determine the times for the formation of the fibrin clot, two different batches of human thrombin preparation were used, one applied to processing batch No. 7006 and the other to No. 7008. N.D.: Not determined | | | | | |

The average results in respect of the solubilisation time of the three processing batches for each of the compositions are compiled in Table 5.

**Table 5**

| Composition (% AMCHA) | Solubilisation time (minutes) (x±SD) (n=3) |
|---|---|
| 0 | >20 |
| 1 | >20 |
| 2 | 8.3±4.5 |
| 2.5 | 9.0±0.6 |
| 3 | 7.7±0.6 |

The above results indicate that the composition of the product with AMCHA reduces the solubilisation time significantly if the concentration of this solubiliser is in the range of from 2% to 3%. The addition of the solubiliser also promotes a significant reduction in the turbidity of the reconstituted solution even at a concentration of 1% (in the metered solution), the minimum value almost being reached when the concentration is 2.5%, or more. The presence of AMCHA does not reduce the gelling time (negative value at 24 hours of the samples in the solution), and the coagulation time with thrombin is somewhat greater in preparations containing AMCHA compared with their control at 0%.

### Example 4

Viral inactivation by heat and its effect on some of the properties of the product are demonstrated in this application example.

The lyophilised vials of the groups of different composition given in the previous Example 3 were used as starting material and were subjected to a thermal treatment effective in inactivating some viruses without a envelope, for which purpose they were introduced into a hot-air oven at a temperature such that the vials concerned reached 100-102°C within some 50 minutes (vial with a control probe), and were left thermostatically controlled in that temperature range for exactly one more hour. Immediately afterwards they were cooled in the surroundings until they reached this final temperature.

The results obtained with respect to the dissolution time of the lyophilisate, the time for the formation of the fibrin clot in the presence of thrombin (some 250-500 IU/ml), the turbidity of the reconstituted lyophilisate and the gelling time, are given in Table 6.

**Table 6**

| Processing batch no. | Composition (% AMCHA) | Solubilisation time (min.) | Coagulation time (sec.) | Turbidity (NTU) | Gelling (at 24 hours) |
|---|---|---|---|---|---|
| 7006 | 0 | >20 (n=2) | 7.5±0.7 (n=2) | 64.2±2.9 (n=2) | YES |
| | 1 | >17 (n=2) | 8.5±0.7 (n=2) | 55.3±0.1(n=2) | NO |
| | 2 | 10±2 (n=2) | 9.0±0.0 (n=2) | 47.1±0.8 (n=2) | NO |
| | 2.5 | 9±1 (n=2) | 11.0±1.4 (n=2) | 40.1±3.4 (n=2) | NO |
| | 3 | 11±4 (n=2) | 13.5±0.7 (n=2) | 44.2±2.5 (n=2) | NO |
| 7007 | 0 | >20 | N.D. | N.D. | YES |
| | 1 | >20 | N.D. | 56.6 | NO |
| | 2 | 14 | 12.5±0.7 (n=2) | 51.0±1.7 (n=2) | NO |
| | 3 | 8 | 12 | 50.8 | NO |
| 7008 | 0 | >20 (n=2) | 12 | N.D. | YES |
| | 1 | >20 (n=2) | N.D. | N.D. | YES |
| | 2 | 13±1 (n=2) | 7.5±0.7 (n=2) | 66.1±0.1 (n=2) | NO |
| | 2.5 | 15±7 (n=2) | 12 | 49.0 | NO |
| | 3 | 15 | 11.5±0.7 (n=2) | 47.7±4A (n=2) | NO |
| Note: (1) The indicated percentage concentration of AMCHA corresponds to the solution before lyophilisation, the metering and reconstitution volume being 10 and 5 ml in each case. (2) In order to determine the times for the formation of the fibrin clot, two different batches of human thrombin preparation were used, one applied to processing batch No. 7006 and the other to No. 7008. N.D.: Not determined | | | | | |

The average values of the solubilisation time of each of the different compositions of all of the prepared batches are compiled in Table 7.

**Table 7**

| Composition (% AMCHA) | Solubilisation time (minutes) (x±SD) (n=3) |
|---|---|
| 0 | >20 |
| 1 | >17 |
| 2 | 12.3±2.1 |
| 2.5 | 12.0±4.2 |
| 3 | 11.3±3.5 |

The results obtained in the finished end product after inactivation by heat confirm the possibility of carrying out this treatment in the final stage, that is to say, when the product has been formulated and packaged. The necessary concentrations of the solubiliser are preferably from 2% to 3% (in the metered solution), since no significant differences between the concentrations with regard to solubilisation time or turbidity of the solubilised material are observed in that range. Likewise, it may be deduced from the results that heating hardly affects the solubilisation time which is prolonged by 3-5 minutes compared with the non-heated product in the formulations having from 2% to 3% of AMCHA.

Heating appears to promote the gelling of the compound, by reducing its useful life in the liquid state, when the concentration of AMCHA is equal to or less than 1%. The turbidity of the product is not increased as a result of heating under the processing conditions described.

## Claims

1. Use of tranexamic acid for the preparation of a composition of human fibrinogen obtained from human plasma or by methods of recombinant or transgenic technology, with the simultaneous addition of human albumin, in order to reduce the solubilisation time when a physiologically compatible solvent is added.

2. A human fibrinogen composition according to claim 1, characterised in that the concentration of tranexamic acid is preferably from 2 to 8% and more preferably from 4 to 5% once the product has been solubilised and in that the composition is virus-inactivated by the action of heat.

3. A human fibrinogen composition according to claim 1, characterised in that the concentration of the majority proteins of the preparation, once solubilised, is from 0.5% to 1.5% in the case of albumin and from 6% to 8% in the case of fibrinogen, and likewise the composition contains not less than 70% of coagulable protein in relation to the total protein.

4. A human fibrinogen composition according to claim 1, characterised in that the concentration of glycine in the solubilised end product is from 0.1M to 0.4M.

5. A human fibrinogen composition according to claim 1, characterised in that the concentration of the saline citrate salts, once the product has been solubilised, is from 0.1M to 0.4M of sodium chloride and from 0.02M to 0.1M of sodium citrate.

6. A human fibrinogen composition according to claim 1, characterised in that virus-inactivation by heat is effected at a temperature of from 80°C to 115°C on the dried end product, with an exposure time of from 5 minutes to 100 hours, and solubilisation in an aqueous medium is carried out in less than 20 minutes, and preferably in from 2 to 15 minutes, at the preferred temperature of from 35°C to 37°C.
